# FASCICULE DE BREVET EUROPEEN

(11) **EP 4 272 708 B1**
(45) Date de publication et mention de la délivrance du brevet: **22.04.2026**
(21) Numéro de dépôt: 23171883.4
(22) Date de dépôt: 05.05.2023
(51) Int. Cl.: A61F 2/40, A61B 17/17

(54) **IMPLANT GLÉNOÏDIEN DE PROTHÈSE D'ÉPAULE INVERSÉE**
INVERSES SCHULTERGELENKPFANNENIMPLANTAT
GLENOIDAL IMPLANT OF AN INVERTED SHOULDER PROSTHESIS

(30) Priorité: 06.05.2022 FR 2204313
(43) Date de publication de la demande: 08.11.2023
(73) Titulaire: FX Shoulder Solutions, 01440 Viriat (FR)
(72) Inventeur: WAHAB, Hassan, 49100 Angers (FR); TORRENS, Carlos, 08860 Castelldefels (ES); SIEGEL, Herrick, Alabama, 35223 (US); SRIKUMARAN, Umasuthan, Maryland, 21042 (US); GRESTA, Giorgio, 42170 Saint Just-Saint Rambert (FR)
(74) Mandataire: LLR

(56) Documents cités:
- WO-A1-2014/075037
- FR-A1- 2 776 506
- FR-A1- 3 048 357
- US-A- 3 842 442
- US-A1- 2014 277 518

## Description

L'invention concerne un implant glénoïdien de prothèse de l'épaule inversée, destiné à être fixé à la cavité glénoïdienne du patient. L'état de la technique le plus proche est le document WO 2014/075037 A1, qui définit le préambule de la revendication 1.

Une prothèse totale de l'épaule inversée comprend un implant huméral, c'est-à-dire une partie fixée dans l'humérus du patient, et un implant glénoïdien, c'est-à-dire une partie fixée sur la cavité glénoïdienne (aussi appelée glène) du patient. Ces deux implants sont articulés l'un avec l'autre afin de restaurer la mobilité du bras du patient opérée.

Dans le cadre d'une prothèse totale de l'épaule dite « inversée », le centre de rotation de l'articulation est déplacé sur l'implant glénoïdien, à l'inverse d'une prothèse dite « anatomique » où il demeure situé sur l'implant huméral.

Cette modification du positionnement du centre de rotation de l'articulation engendre une modification des muscles sollicités pour mouvoir le bras. En l'espèce, c'est le muscle deltoïde qui est sollicité tandis que pour une prothèse anatomique, les muscles sollicités demeurent ceux formant la coiffe des rotateurs, à savoir les muscles : sous-scapulaire, sus-épineux, sous-épineux et petit rond en plus du deltoïde. Cette modification des muscles impliqués peut s'expliquer par un endommagement irréparable de tout ou partie des muscles de la coiffe des rotateurs, un tel endommagement pouvant intervenir chez les personnes âgées notamment. En d'autres termes, et pour les personnes atteintes d'un endommagement irréparable au niveau des muscles formant la coiffe des rotateurs, une prothèse d'épaule inversée est préférée car elle permet de conserver une mobilité de l'épaule dès les premiers degrés d'abduction du bras par rapport au tronc. La prothèse d'épaule inversée implique donc la sollicitation du muscle deltoïde qui est ancré sur l'humérus de manière plus distale que les muscles de la coiffe des rotateurs, elle compense alors l'endommagement des muscles formant la coiffe des rotateurs.

La prothèse d'épaule inversée est formée par un implant huméral comprenant une extrémité creuse (ou cupule) remplaçant la tête humérale et, au niveau de l'omoplate, une embase d'ancrage osseux (ou métaglène) sur laquelle est rapportée une glénosphère destinée à collaborer avec la cupule positionnée sur l'humérus.

Classiquement, l'embase d'ancrage osseux présent au niveau de l'omoplate est fixée à cet os par l'intermédiaire de plusieurs vis d'ancrage s'étendant à partir de l'embase d'ancrage osseux et divergentes. Cet éloignement permet d'assurer un bon ancrage de l'implant glénoïdien en sollicitant des points de fixation éloignés.

Cependant, les prothèses d'épaule, et plus particulièrement les prothèses d'épaule inversées, sont utilisées pour restaurer l'articulation de personnes souffrant d'arthrose, notamment la prothèse d'épaule inversée pour les raisons de mobilité de l'épaule expliquées ci-dessus. A un stade avancé d'arthrose, la dégradation de l'articulation, et plus particulièrement de la glène de l'omoplate, peut-être trop importante pour envisager un ancrage stable de l'implant glénoïdien classique. En d'autres termes, la surface osseuse disponible ou capital osseux n'est plus suffisante (l'os se creusant), notamment au niveau de la portion reliant la glène à la scapula, pour assurer un ancrage stable de l'embase d'ancrage osseux, ce qui peut remettre en cause la possibilité de restauration de l'articulation par une prothèse d'épaule, même inversée.

L'invention a pour but de fournir un implant glénoïdien pour prothèse d'épaule inversée permettant l'utilisation d'une prothèse d'épaule pour les personnes souffrant d'arthrose à un stade avancé et pour lesquelles il ne serait pas possible de prévoir l'utilisation d'une prothèse selon l'art antérieur.

A cet effet, l'invention a pour objet un implant glénoïdien de prothèse d'épaule inversée tel que défini dans la revendication 1.

Ainsi, et au lieu d'utiliser une embase d'ancrage difficile à stabiliser, la tige s'étend dans l'épaisseur de l'os, dans une travée osseuse, et est stabilisée dans l'os par des vis d'ancrage traversant radialement la tige et l'os. On assure donc un ancrage stable de l'implant glénoïdien avec une structure s'étendant moins (une tige) en exploitant des zones osseuses moins atteintes, ce qui est particulièrement adapté aux personnes souffrant d'arthrose à un stade avancé.

Suivant d'autres caractéristiques optionnelles de l'implant glénoïdien prises seules ou en combinaison :
- L'implant glénoïdien comprend une glénosphère rapportée sur la tige ;
- la tige comprend une surface de réception d'une entretoise de fixation de la tige à la glénosphère ;
- la surface de réception comprend un alésage configuré pour collaborer avec l'entretoise et une gorge entourant l'alésage, la gorge étant configurée pour interagir avec un outil de préhension et de guidage de la tige ;
- l'entretoise est formée d'une première portion de fixation de l'entretoise à la tige et d'une seconde portion de fixation de l'entretoise à la glénosphère, la première portion et la seconde portion étant décalées l'une par rapport à l'autre ;
- la première portion et la seconde portion sont inclinées l'une par rapport à l'autre ;
- l'implant glénoïdien comprend en outre une pièce stabilisatrice de l'implant glénoïdien par rapport à l'os lorsque l'implant glénoïdien est implanté sur l'os ; et
- la pièce stabilisatrice comprend deux extrémités de stabilisation configurées pour s'étendre de part et d'autre de l'os lorsque l'implant glénoïdien est implanté sur l'os.

L'invention a également pour objet une prothèse d'épaule inversée comprenant un implant glénoïdien selon l'invention.

L'invention a également pour objet un kit comprenant un outil de préhension et de guidage d'un implant glénoïdien selon l'invention tel que défini dans la revendication 10.

Suivant d'autres caractéristiques optionnelles de l'implant glénoïdien prises seules ou en combinaison :
- l'outil de préhension et de guidage comprend au moins un élément de guidage pour la mise en place des vis d'ancrage amovible par rapport à l'extrémité de préhension et pouvant s'étendre entre le trou de guidage et un orifice traversant ; et
- l'extrémité de préhension est mobile en rotation par rapport à l'extrémité de verrouillage.

L'invention a également pour objet une utilisation d'un implant glénoïdien selon l'invention pour former une prothèse articulaire.

### Brève description des figures

L'invention sera mieux comprise à la lecture de la description qui va suivre donnée uniquement à titre d'exemple et faite en se référant aux dessins annexés dans lesquels :
[Fig. 1] est une vue en perspective d'un implant glénoïdien de prothèse d'épaule inversée selon l'invention,
[Fig. 2] est une vue de côté d'un implant glénoïdien de prothèse d'épaule inversée selon l'invention,
[Fig. 3] est une vue en perspective d'un implant glénoïdien de prothèse d'épaule inversée selon l'invention placé au niveau d'une omoplate,
[Fig. 4] est une vue en perspective d'une tige d'un implant glénoïdien de prothèse d'épaule inversée selon l'invention,
[Fig. 5] est une vue en perspective d'une première variante d'une entretoise d'un implant glénoïdien de prothèse d'épaule inversée selon l'invention,
[Fig. 6] est une vue de côté d'une seconde variante d'une entretoise d'un implant glénoïdien de prothèse d'épaule inversée selon l'invention,
[Fig. 7] est une vue en perspective d'une seconde variante d'une entretoise d'un implant glénoïdien de prothèse d'épaule inversée selon l'invention,
[Fig. 8] est une vue de côté d'une seconde variante d'une entretoise d'un implant glénoïdien de prothèse d'épaule inversée selon l'invention,
[Fig. 9] est une vue en perspective d'une pièce stabilisatrice d'un implant glénoïdien de prothèse d'épaule inversée selon l'invention,
[Fig. 10] est une vue en perspective d'une première variante d'une glénosphère d'un implant glénoïdien de prothèse d'épaule inversée selon l'invention,
[Fig. 11] est une vue en perspective d'une seconde variante d'une glénosphère d'un implant glénoïdien de prothèse d'épaule inversée selon l'invention, et
[Fig. 12] est une vue en perspective d'une tige d'un implant glénoïdien de prothèse d'épaule inversée selon l'invention attachée à un outil de préhension et de guidage de cette dernière,
[Fig. 13] est une vue éclatée d'une tige d'un implant glénoïdien de prothèse d'épaule inversée selon l'invention et d'une partie d'un outil de préhension et de guidage de la tige,
[Fig. 14] est une vue en perspective d'une tige d'un implant glénoïdien de prothèse d'épaule inversée selon l'invention en cours de liaison à un outil de préhension et de guidage de la tige, et
[Fig. 15] est une vue en perspective d'un implant glénoïdien en cours d'implantation sur une omoplate.

### Description détaillée

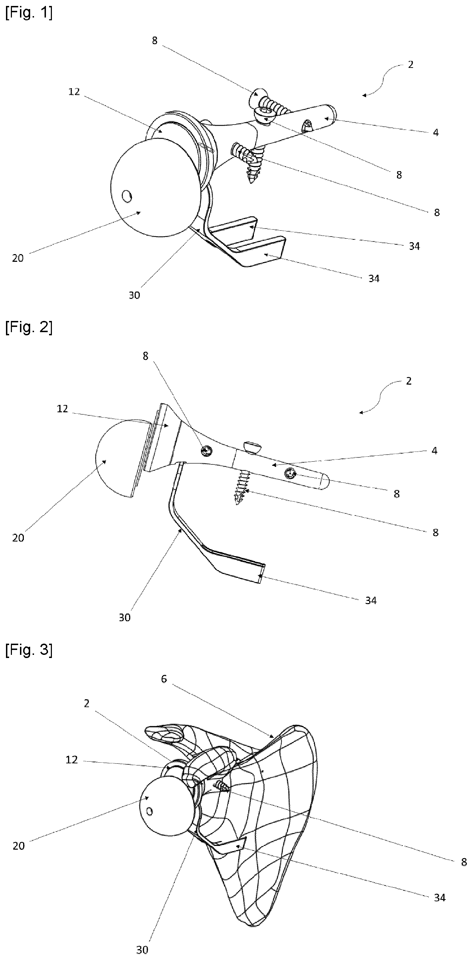

On se réfère désormais aux figures 1 et 2 illustrant un implant glénoïdien 2 de prothèse d'épaule inversée.

Cet implant glénoïdien 2 comprend une embase d'ancrage formée par une tige 4 configurée pour s'étendre dans l'épaisseur d'un os 6 (une omoplate visible sur la figure 3) depuis une cavité glénoïde et par des vis d'ancrage 8 (ou vis corticales) de la tige 4 dans l'os configurées pour traverser radialement la tige 4 et l'os 6. Comme expliqué plus haut, la tige 4 s'étend dans une travée osseuse de l'os 6, par exemple au niveau de la jonction entre la scapula et l'acromion, pour permettre un ancrage, par mise en place des vis d'ancrage 8, sans trop s'étendre dans l'os 6, ce qui pourrait poser problème en cas d'arthrose à un stade trop avancé. On comprend donc que le chirurgien va procéder à la réalisation d'un alésage dans l'épaisseur de l'os 6 pour y loger la tige 4. Les vis d'ancrage 8 sont ensuite mises en place pour traverser radialement l'os 6 et la tige 4 (par des orifices traversants 18 de cette dernière) et ainsi verrouiller la tige 4 dans l'os 6 comme illustré sur la figure 3. On comprend bien évidemment que « radialement » signifie selon une direction perpendiculaire à un axe longitudinal A de la tige 4.

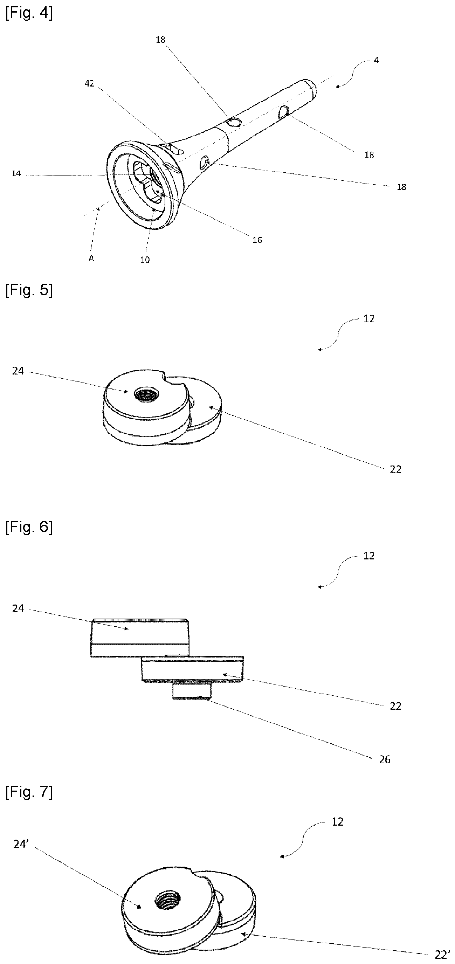

La tige 4, visible seule sur la figure 4, est réalisée dans un matériau biocompatible et ayant une durée de vie optimale la plus longue possible. Elle peut par exemple être réalisée en alliage de titane, en acier inoxydable, en chrome cobalt ou encore à partir d'un matériau polymère. La longueur de la tige 4 peut varier afin de s'adapter au mieux à la taille de l'os 6, avec un diamètre pouvant varier entre 5 et 14 millimètres. Il est possible à cet effet de concevoir plusieurs tiges 4 de tailles variables et longueur variable ou une tige 4 formée par un assemblage de plusieurs parties (chaque partie peut par exemple portée au moins un orifice traversant 18) dont les longueurs peuvent varier afin d'assembler une tige 4 adaptée à l'os 6. La longueur de la tige 4 peut par exemple varier entre 40 et 120 millimètres.

La tige 4 peut comprendre une face de réception 10 d'une entretoise 12, la face de réception 10 pouvant comprendre un alésage 14, par exemple taraudé, permettant la fixation de l'entretoise 12 via une vis et ou un cône ou un clip, par exemple par vissage si l'alésage 14 est taraudé, potentiellement complété par une gorge 16 formant une surface d'interaction avec un outil de préhension et de guidage 36 décrit plus bas.

La tige 4 comprend en outre des orifices traversants 18 permettant le passage des vis d'ancrage 8, ici au nombre de trois. De préférence, les orifices traversants 18 sont réalisés au niveau des zones de la tige 4 qui seront localisées au niveau des zones de l'os 6 où l'épaisseur de ce dernier sera la plus importante. Ainsi, les vis d'ancrage 8 traverseront une épaisseur d'os 6 la plus importante possible. Il est par ailleurs possible de verrouiller la tige 4 en sollicitant l'acromion et la coracoïde.

Comme entrevu ci-dessus, la tige 4 peut recevoir une entretoise (ou bague rapportée) 12. Cette dernière permet d'assurer une liaison à distance entre la tige 4 et la glénosphère 20.

Cette entretoise 12 peut être formée d'un seul tenant ou par plusieurs parties assemblées l'une à l'autre. Elle peut être totalement coaxiale de la tige 4 ou présenter un axe de fixation de la glénosphère 20 décalé par rapport à l'axe de fixation de l'entretoise 12 sur la tige 4. Ces axes de fixation peuvent être parallèles entre eux ou non. Ces différentes configurations permettent d'adapter l'implant glénoïdien 2 au patient la recevant. L'entretoise 12 est rapportée sur la tige 4 après que cette dernière ait été verrouillée sur l'os 6.

Les figures 5 et 6 illustrent une première variante d'une entretoise 12. Cette dernière est formée de deux portions 22 et 24 réalisée d'un seul tenant mais décalées, par exemple de 1 à 25 millimètres l'une par rapport à l'autre. La première portion 22, permettant de relier la tige 4 à l'entretoise 12, comporte un premier orifice permettant d'effectuer cette liaison, par exemple par vissage en étant fileté et/ou conique. Cet orifice s'aligne avec l'alésage 14 de la tige 4. La seconde portion 24, permettant de relier la glénosphère 20 à l'entretoise 12, comporte un second orifice permettant d'effectuer cette liaison, par exemple par vissage en étant taraudé et en collaborant avec une saillie 28 de la glénosphère 20 comprenant un filetage externe. La fixation peut également être réalisée en utilisant un cône morse. Les portions sont parallèles entre elles.

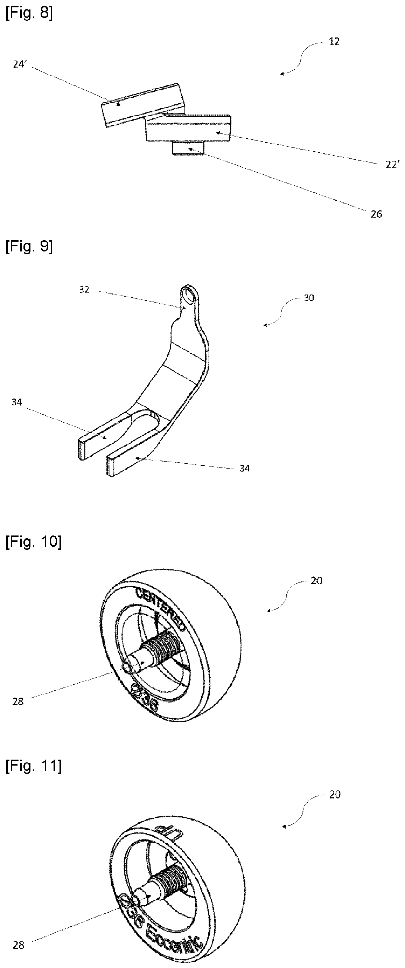

Les figures 7 et 8 illustrent une seconde variante d'une entretoise 12. Cette dernière est formée de deux portions 22' et 24' réalisée d'un seul tenant mais décalées, par exemple de 1 à 25 millimètres l'une par rapport à l'autre. La première portion 22', permettant de relier la tige 4 à l'entretoise 12, comporte un premier orifice permettant d'effectuer cette liaison, par exemple par vissage en étant fileté. Cet orifice s'aligne avec l'alésage 14 de la tige 4. Cette dernière porte une protubérance 26 collaborant avec la gorge 16 comme expliqué ci-dessus. La seconde portion 24', permettant de relier la glénosphère 20 à l'entretoise 12, comporte un second orifice permettant d'effectuer cette liaison par exemple par vissage en étant fileté et en collaborant avec une saillie 28 de la glénosphère 20 comprenant un filetage externe. Les portions sont inclinées l'une par rapport à l'autre, par exemple en formant un angle compris entre 5° et 30°, ce qui permet de fixer la glénosphère 20 de manière inclinée par rapport à la tige 4.

Comme pour la tige 4, l'entretoise 12 est réalisée dans un matériau biocompatible et ayant une durée de vie optimale la plus longue possible, permettant une bonne répartition des contraintes mécaniques. L'entretoise 12 peut notamment être réalisée tout ou partie en métal, en plastique ou en céramique. Elle peut également être réalisée avec plusieurs matériaux différents dont une composition des matériaux précités.

L'entretoise 12 est dans les exemples décrits une pièce rapportée sur la tige 4. Elle pourrait également être réalisée d'un seul tenant avec cette dernière et formée une extrémité de la tige 4.

La glénosphère 20 est illustrée sur les figures 10 et 11. Cette glénosphère 20 peut être de forme sphérique (demi-sphère, portion de sphère supérieure à une demi-sphère, portion de sphère prolongée par une portion cylindrique). Son diamètre peut être compris entre 30 et 46 millimètres. Ce diamètre peut bien évidemment être en dehors de cet intervalle. Elle peut être réalisée en céramique. Le type de céramique utilisé peut être tout type de céramique connu de l'homme du métier. A titre d'exemple, il peut s'agir de l'alumine, de la zircone ou d'un matériau composite. La céramique étant un matériau de type dur, il peut être utilisé dans le cadre de couple de matériaux « dur/dur » ou « dur-mou ». Dans ce second exemple, le matériau dit « mou » peut être du polyéthylène de masse molaire très élevée (UHMWPE), du polyétheréthercétone (PEEK), du PEKK(poly ether ketone ketone) ou leurs dérivés.

La glénosphère 20 peut être centrée ou non et peut être latéralisée le cas échéant (comme vu ci-dessus, notamment en fonction de la forme de l'entretoise 12). Un centrage signifie qu'une saillie 28 permettant la fixation de la glénosphère 20 à l'entretoise 12 s'étend à partir du centre de la surface plane de l'hémisphère dans une direction perpendiculaire au plan dans lequel s'étend la surface plane de l'hémisphère. Un excentrage, par exemple compris entre 1 et 4 millimètres) signifie qu'une saillie 28 permettant la fixation de la glénosphère 20 à l'entretoise 12 ne s'étend pas à partir du centre de la surface plane de l'hémisphère. La saillie 28 peut comprendre un filetage externe permettant de visser la glénosphère 20 sur l'entretoise 12, plus particulièrement dans l'orifice de la seconde portion 24 ou 24' de l'entretoise 12, fileté dans ce cas de figure comme vu ci-dessus.

La glénosphère 20 peut être marquée, par exemple par gravure, pour indiquer son diamètre, le fait qu'elle soit centrée ou excentrique, ou encore, et si nécessaire, pour inscrire une mention permettant de repérer la portion de la glénosphère 20 devant être placée angulairement vers la position supérieure de l'omoplate, à savoir "UP" dans l'exemple représenté sur la figure 11 de la glénosphère excentrique.

La possibilité d'utilisation d'une glénosphère 20 de diamètre variable, centrée ou excentrée, permet une possibilité d'adaptation supplémentaire de l'implant glénoïdien 2 au patient, en complément du choix de la forme de l'entretoise 12 et de la taille de la tige 4.

L'implant glénoïdien 2 peut en outre comprendre une pièce stabilisatrice 30 (ou fourchette stabilisatrice). Cette dernière, visible seule sur la figure 9 ou montée sur la tige 4 sur les figures 1 à 3, peut être rapportée sur la tige 4, qui comprend dès lors une fente de fixation 42 de la pièce stabilisatrice 30. Plus précisément, la fente de fixation 42 peut déboucher au niveau d'un taraudage interne de la tige 4. Une pièce intermédiaire comprenant un filetage externe peut dès lors être vissée à la tige 4 et verrouiller la pièce stabilisatrice 30.

Cette pièce stabilisatrice 30 peut comprendre une extrémité de verrouillage 32, avantageusement percée, de la pièce stabilisatrice 30 à la tige 4 et deux extrémités de stabilisation 34. Ces dernières sont disposées en regard d'une de l'autre de sorte à s'étendre de part et d'autre de l'os 6 (ici de la scapula) lorsque l'implant glénoïdien 2 est mis en place. On peut envisager qu'au moins une extrémité de stabilisation 34 soit en appui contre l'os 6 lorsque l'implant glénoïdien 2 est mis en place. Elle peut dans ce cas avoir une surface plane s'étendant parallèlement à l'os 6 afin d'obtenir un appui sur une surface importante. Le nombre et la forme des extrémités de stabilisation 34 peut varier.

La pièce stabilisatrice 30 pourrait également permettre un ancrage osseux supplémentaire pour l'implant glénoïdien 2. Au moins une extrémité de stabilisation 34 (ou l'extrémité de stabilisation s'il n'y en a qu'une) peut dans ce cas comprendre un orifice traversant pour la fixer à l'os 6, par exemple par vissage et ainsi créer un point d'ancrage supplémentaire.

Comme vu avant pour d'autres éléments, la pièce stabilisatrice 30 est réalisée dans un matériau biocompatible, ayant une durée de vie optimale la plus longue possible et permettant une bonne reprise des efforts. Elle peut par exemple être réalisée en alliage de titane, en acier inoxydable, en chrome cobalt ou encore à partir d'un matériau polymère.

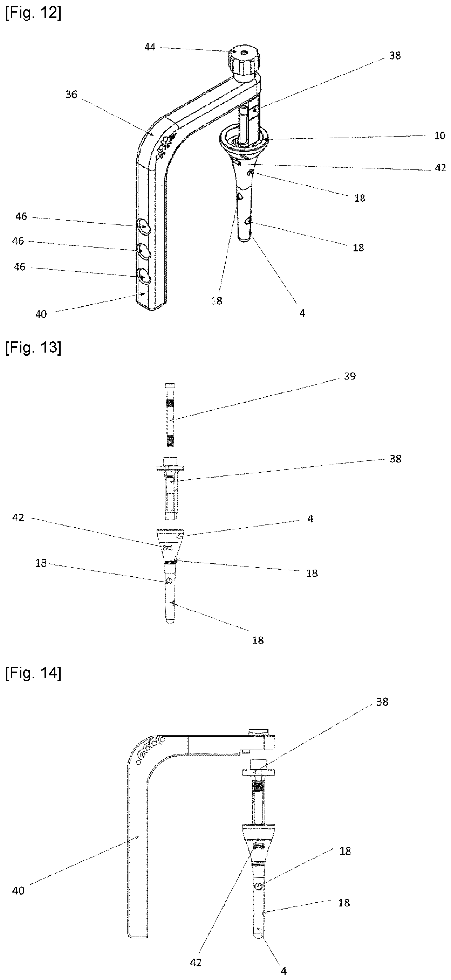

La figure 12 illustre quant à elle la tige 4 attachée à un outil de préhension et de guidage 36. Ce dernier comprend une extrémité de verrouillage 38 configurée pour collaborer avec la gorge 16 et l'alésage 14 afin d'attacher la tige 4 à l'outil de préhension et de guidage 36 et de pouvoir placer cette dernière dans la cavité ménagée dans l'os 6. L'outil de préhension et de guidage 36 comprend également une extrémité de préhension 40 de l'ensemble formé par ce dernier et la tige 4 par un chirurgien.

L'extrémité de verrouillage 38 et l'extrémité de préhension 40 sont avantageusement deux parties séparées de l'outil de préhension et de guidage 36. Dès lors, l'extrémité de verrouillage 38 est dans un premier temps rapportée sur la tige 4 (que cette dernière soit munie ou non de la pièce stabilisatrice 30). Cette extrémité de verrouillage 38 est avantageusement creuse et partiellement taraudée de manière à y insérer une pièce intermédiaire 39 comprenant au moins un filetage externe de manière à verrouiller par vissage l'extrémité de verrouillage 38 sur la tige 4 (voir figure 13).

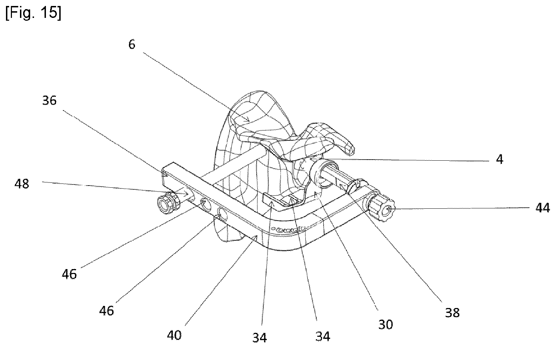

L'extrémité de préhension 40 est ensuite rapportée sur l'extrémité de verrouillage 38 (voir figure 14) et fixée à cette dernière, par exemple en les vissant l'une à l'autre, par exemple par une vis à tête moletée 44 visible sur les figures 12 et 15.

L'extrémité de préhension 40 comprend une partie s'étendant parallèlement à la tige 4 lorsque cette dernière est tenue par l'outil de préhension et de guidage 36 et comprend des trous 46 pouvant être alignés avec les orifices traversants 18. Ces trous de guidage 46 permettent de guider un chirurgien lors de la mise en place de la tige 4. En effet, l'alignement lui permet de savoir où percer l'os 6 pour retrouver un orifice traversant 18. Il peut pour cela positionner un élément de guidage 48, ici de forme cylindrique, s'étendant à partir des trous de guidage 46 et perpendiculairement à la tige 4 et à la partie de l'extrémité de préhension 40 portant les trous de guidage 46 (voir figure 15). Cet élément de guidage 48 vient dès lors en butée contre l'os 6 et est avantageusement creux de manière à pouvoir y introduire des moyens de perçage de l'os 6 puis les vis d'ancrage 8 et des moyens de vissage de ces dernières à la tige 4. Les vis d'ancrage 8 traversent dès lors l'os 6 et la tige 4.

Sur la figure 15, on peut voir une tige 4, portant une pièce stabilisatrice 30, déjà en place dans une cavité ménagée dans l'épaisseur de l'os 6. L'extrémité de préhension 40 est placée de telle sorte à aligner au moins un trou de guidage 46 avec un orifice traversant 18 (non visible sur cette figure). Un élément de guidage 48 s'étend depuis un trou 46 et est plaqué contre l'os 6.

Comme cela est visible sur la figure 4, les orifices traversants 18 peuvent ne pas être alignés mais s'étendre dans des directions différentes pour au moins deux d'entre eux. Il est dès lors avantageux de prévoir une capacité de rotation de l'extrémité de préhension 40 par rapport à l'extrémité de verrouillage 38. Ainsi, il est possible de déplacer, l'extrémité de préhension 40 pour aligner un trou de guidage 46 avec un orifice traversant 18. Sur la figure 4, deux orifices traversants 18 sont alignés et le troisième décalé de 90°. L'extrémité de préhension 40 peut dès lors pivoter de 90° pour s'aligner avec un orifice traversant 18 ou un autre.

### Liste de références

2 : implant glénoïdien
4 : tige
6 : os
8 : vis d'ancrage
10 : surface de réception
12 : entretoise
14 : alésage
16 : gorge
18 : orifices traversants
20 : glénosphère
22, 22' : premières portions de l'entretoise
24, 24' : secondes portions de l'entretoise
26 : protubérance
28 : saillie
30 : pièce stabilisatrice
32 : extrémité de verrouillage
34 : extrémité de stabilisation
36 : outil de préhension et de guidage
38 : extrémité de verrouillage
39 : pièce intermédiaire
40 : extrémité de préhension
42 : fente de fixation
44 : vis à tête moletée
46 : trous de guidage
48 : élément de guidage
A : axe longitudinal de la tige

## Revendications

1. Implant glénoïdien (2) de prothèse d'épaule inversée, comprenant une embase d'ancrage de l'implant à un os formée par une tige (4) configurée pour s'étendre dans l'épaisseur de l'os (6) depuis une cavité glénoïde, la tige (4) comprenant des orifices traversants (18), et par des vis d'ancrage (8) de la tige (4) configurées pour traverser radialement la tige (4), par les orifices traversants (18), et l'os (6), lorsque l'implant glénoïdien (2) est implanté sur l'os (6), **caractérisé en ce que** la tige (4) est formée de plusieurs parties amovibles les unes par rapport aux autres de manière à pouvoir former une tige de longueur variable, par exemple comprise entre 40 et 120 millimètres.

2. Implant glénoïdien (2) selon la revendication 1, comprenant une glénosphère (20) rapportée sur la tige (4).

3. Implant glénoïdien (2) selon l'une quelconque des revendications précédentes, dans lequel la tige (4) comprend une surface de réception (10) d'une entretoise (12) de fixation de la tige (4) à la glénosphère (20).

4. Implant glénoïdien (2) selon la revendication précédente, dans lequel la surface de réception (10) comprend un alésage (14) configuré pour collaborer avec l'entretoise (12) et une gorge (16) entourant l'alésage (14), la gorge (16) étant configurée pour interagir avec un outil de préhension et de guidage (34) de la tige (4).

5. Implant glénoïdien (2) selon l'une quelconque des revendications 3 ou 4, dans lequel l'entretoise (12) est formée d'une première portion (22, 22') de fixation de l'entretoise (12) à la tige (4) et d'une seconde portion (24, 24') de fixation de l'entretoise (12) à la glénosphère (20), la première portion (22, 22') et la seconde portion (24, 24') étant décalées l'une par rapport à l'autre.

6. Implant glénoïdien (2) selon la revendication précédente, dans lequel la première portion (22, 22') et la seconde portion (24, 24') sont inclinées l'une par rapport à l'autre.

7. Implant glénoïdien (2) selon l'une quelconque des revendications précédentes, comprenant en outre une pièce stabilisatrice (30) de l'implant glénoïdien (2) par rapport à l'os (6) lorsque l'implant glénoïdien (2) est implanté sur l'os (6).

8. Implant glénoïdien (2) selon la revendication précédente, dans lequel la pièce stabilisatrice (30) comprend deux extrémités de stabilisation (34) configurées pour s'étendre de part et d'autre de l'os (6) lorsque l'implant glénoïdien (2) est implanté sur l'os (6).

9. Prothèse d'épaule inversée comprenant un implant glénoïdien (2) selon l'une quelconque des revendications 1 à 8.

10. Kit comprenant un outil de préhension et de guidage (36) d'un implant glénoïdien (2) selon l'une quelconque des revendications 1 à 8 et un implant glénoïdien (2) selon l'une quelconque des revendications 1 à 8, l'outil de préhension et de guidage (36) comprenant une extrémité de verrouillage (38) à la tige (4) et une extrémité de préhension (40), l'extrémité de préhension (40) s'étendant au moins en partie parallèlement à la tige (4) lorsque l'outil de préhension et de guidage (36) est fixé à la tige (4), l'extrémité de préhension (40) comprenant au moins un trou de guidage (46) pour la mise en place des vis d'ancrage (8), l'outil de préhension et de guidage (36) comprenant au moins un élément de guidage (48) pour la mise en place des vis d'ancrage (8) amovible par rapport à l'extrémité de préhension (40) et pouvant s'étendre entre le trou de guidage (46) et un orifice traversant (18).

11. Kit selon la revendication 10, dans lequel l'extrémité de préhension (40) est mobile en rotation par rapport à l'extrémité de verrouillage (38).

## Patentansprüche

1. Schultergelenkpfannen-Implantat (2) für inverse Schulterprothese, umfassend eine Verankerungsbasis zur Verankerung des Implantats an einem Knochen, die von einem Schaft (4) gebildet ist, der so ausgebildet ist, dass er sich in der Dicke des Knochens (6) von einer Schultergelenkpfanne aus erstreckt, wobei der Schaft (4) Durchgangsöffnungen (18) umfasst, und durch Verankerungsschrauben (8) zur Verankerung des Schafts (4), die so ausgebildet sind, dass sie den Schaft (4), über die Durchgangsöffnungen (18), und den Knochen (6) radial durchqueren, wenn das Schultergelenkpfannen-Implantat (2) an dem Knochen (6) implantiert ist, **dadurch gekennzeichnet, dass** der Schaft (4) von mehreren Teilen gebildet ist, die relativ zueinander so entfernbar sind, dass sie einen Schaft variabler Länge bilden können, zum Beispiel zwischen 40 und 120 Millimeter.

2. Schultergelenkpfannen-Implantat (2) nach Anspruch 1, umfassend eine Glenosphäre (20), die an dem Schaft (4) angebracht ist.

3. Schultergelenkpfannen-Implantat (2) nach einem der vorhergehenden Ansprüche, wobei der Schaft (4) eine Aufnahmefläche (10) zur Aufnahme eines Distanzstücks (12) zur Befestigung des Schafts (4) an der Glenosphäre (20) umfasst.

4. Schultergelenkpfannen-Implantat (2) nach dem vorhergehenden Anspruch, wobei die Aufnahmefläche (10) eine Bohrung (14) für das Zusammenwirken mit dem Distanzstück (12) und eine Rille (16), welche die Bohrung (14) umgibt, umfasst, wobei die Rille (16) für das Zusammenwirken mit einem Greif- und Führungswerkzeug (34) zum Greifen und Führen des Schafts (4) ausgebildet ist.

5. Schultergelenkpfannen-Implantat (2) nach einem der Ansprüche 3 oder 4, wobei das Distanzstück (12) von einem ersten Abschnitt (22, 22') zur Befestigung des Distanzstücks (12) an dem Schaft (4) und einem zweiten Abschnitt (24, 24') zur Befestigung des Distanzstücks (12) an der Glenosphäre (20) gebildet ist, wobei der erste Abschnitt (22, 22') und der zweite Abschnitt (24, 24') relativ zueinander versetzt sind.

6. Schultergelenkpfannen-Implantat (2) nach dem vorhergehenden Anspruch, wobei der erste Abschnitt (22, 22') und der zweite Abschnitt (24, 24') relativ zueinander geneigt sind.

7. Schultergelenkpfannen-Implantat (2) nach einem der vorhergehenden Ansprüche, ferner umfassend ein Stabilisierungsteil (30) zur Stabilisierung des Schultergelenkpfannen-Implantats (2) gegenüber dem Knochen (6), wenn das Schultergelenkpfannen-Implantat (2) an dem Knochen (6) implantiert ist.

8. Schultergelenkpfannen-Implantat (2) nach dem vorhergehenden Anspruch, wobei das Stabilisierungsteil (30) zwei Stabilisierungsenden (34) umfasst, die so ausgebildet sind, dass sie sich auf beiden Seiten des Knochens (6) erstrecken, wenn das Schultergelenkpfannen-Implantat (2) an dem Knochen (6) implantiert ist.

9. Inverse Schulterprothese, umfassend ein Schultergelenkpfannen-Implantat (2) nach einem der Ansprüche 1 bis 8.

10. Kit, umfassend ein Greif- und Führungswerkzeug (36) zum Greifen und Führen eines Schultergelenkpfannen-Implantats (2) nach einem der vorhergehenden Ansprüche 1 bis 8 und ein Schultergelenkpfannen-Implantat (2) nach einem der Ansprüche 1 bis 8, wobei das Greif- und Führungswerkzeug (36) ein Verriegelungsende (38) zur Verrieglung an dem Schaft (4) und ein Greifende (40) umfasst, wobei das Greifende (40) sich mindestens teilweise parallel zu dem Schaft (4) erstreckt, wenn das Greif- und Führungswerkzeug (36) an dem Schaft (4) befestigt ist, wobei das Greifende (40) mindestens ein Führungsloch (46) zum Einsetzen der Verankerungsschrauben (8) umfasst, wobei das Greif- und Führungswerkzeug (36) mindestens ein Führungselement (48) zum Einsetzen der Verankerungsschrauben (8) umfasst, das relativ zu dem Greifende (40) entfernbar ist und sich zwischen dem Führungsloch (46) und einer Durchgangsöffnung (18) erstrecken kann.

11. Kit nach Anspruch 10, wobei das Greifende (40) relativ zu dem Verriegelungsende (38) drehbeweglich ist.

## Claims

1. Glenoid implant (2) of a reverse shoulder prosthesis, comprising an anchoring base for anchoring the implant to a bone formed by a stem (4) configured to extend within the thickness of the bone (6) from a glenoid cavity, the stem (4) comprising through-holes (18), and by anchoring screws (8) for anchoring the stem (4) configured to extend radially through the stem (4), through the through-holes (18), and the bone (6), when the glenoid implant (2) is implanted on the bone (6),
**characterised in that** the stem (4) is formed of several parts removable relative to one another so as to be able to form a stem of variable length, for example between 40 and 120 millimetres.

2. Glenoid implant (2) according to claim 1, comprising a glenosphere (20) mounted on the stem (4).

3. Glenoid implant (2) according to any one of the preceding claims, wherein the stem (4) comprises a receiving surface (10) for receiving a spacer (12) for fastening the stem (4) to the glenosphere (20).

4. Glenoid implant (2) according to the preceding claim, wherein the receiving surface (10) comprises a bore (14) configured to cooperate with the spacer (12) and a groove (16) surrounding the bore (14), the groove (16) being configured to interact with a gripping and guiding tool (34) of the stem (4).

5. Glenoid implant (2) according to any one of claims 3 or 4, wherein the spacer (12) is formed of a first portion (22, 22') for fastening the spacer (12) to the stem (4) and a second portion (24, 24') for fastening the spacer (12) to the glenosphere (20), the first portion (22, 22') and the second portion (24, 24') being offset relative to one another.

6. Glenoid implant (2) according to the preceding claim, wherein the first portion (22, 22') and the second portion (24, 24') are inclined relative to one another.

7. Glenoid implant (2) according to any one of the preceding claims, further comprising a stabilising member (30) for stabilising the glenoid implant (2) relative to the bone (6) when the glenoid implant (2) is implanted on the bone (6).

8. Glenoid implant (2) according to the preceding claim, wherein the stabilising member (30) comprises two stabilising ends (34) configured to extend on either side of the bone (6) when the glenoid implant (2) is implanted on the bone (6).

9. Reverse shoulder prosthesis comprising a glenoid implant (2) according to any one of claims 1 to 8.

10. Kit comprising a gripping and guiding tool (36) for a glenoid implant (2) according to any one of claims 1 to 8 and a glenoid implant (2) according to any one of claims 1 to 8, the gripping and guiding tool (36) comprising a locking end (38) for locking to the stem (4) and a gripping end (40), the gripping end (40) extending at least partly parallel to the stem (4) when the gripping and guiding tool (36) is fixed to the stem (4), the gripping end (40) comprising at least one guiding hole (46) for positioning the anchoring screws (8), the gripping and guiding tool (36) comprising at least one guiding element (48) for positioning the anchoring screws (8), removable relative to the gripping end (40) and capable of extending between the guiding hole (46) and a through-hole (18).

11. Kit according to claim 10, wherein the gripping end (40) is rotatable relative to the locking end (38).
